# EUROPEAN PATENT APPLICATION

(11) **EP 4 198 268 A1**
(43) Date of publication of application: **21.06.2023**
(21) Application number: 22212337.4
(22) Date of filing: 08.12.2022
(51) Int. Cl.: F01D 21/00, A61B 1/00, B25B 23/00, G02B 23/24

(54) **SYSTEM AND METHOD OF ADJUSTING A SEGMENTED TOOL**

(30) Priority: 16.12.2021 US 202117552848
(71) Applicant: Oliver Crispin Robotics Limited, Altrincham, Cheshire WA14 2DT (GB)
(72) Inventor: GRAHAM, Andrew Crispin, Bristol, BS34 7JU (GB); HAWKE, Trevor Owen, Bristol, BS34 7JU (GB); NISBET, Peter John, Brisol, BS34 7JU (GB)
(74) Representative: Openshaw & Co.

(57) **Abstract**

The present disclosure relates to a method (200) for adjusting a tool (100) insertable into a cavity of a machine including a plurality of segments (102), where two adjacent segments (102) are moveable between a bent position and a coupled position. The method (200) includes determining an initial alignment measurement (160) of the two adjacent segments (102) in the coupled position; comparing the initial alignment measurement (160) to a target alignment measurement (165) to determine an adjustment amount; and adjusting at least one of the two adjacent segments (102) based on the adjustment amount. The tool (100) also includes a unique dimensional measurement affecting a fit tolerance with the adjacent segment (102) when in the coupled position, and each segment (102) at least partially comprises an adjustable material (162).

## Description

### FIELD OF THE DISCLOSURE

The present subject matter relates generally to a system and method of adjusting a tool for inspecting an environment and/or performing maintenance operations on a component within the environment, such as within a turbine engine.

### BACKGROUND OF THE PRESENT DISCLOSURE

In a variety of industries, tools, e.g., insertion tools, are used to detect damaged or deteriorated components. For example, in the aviation industry, certain gas turbine engines include thousands of internal components, including hundreds of compressor and turbine blades, which need to be frequently inspected and/or maintained to ensure they are in working order and not damaged.

### BRIEF DESCRIPTION OF THE DRAWINGS

A full and enabling disclosure of the present disclosure, including the best mode thereof, directed to one of ordinary skill in the art, is set forth in the specification, which makes reference to the appended figures, in which:
FIG. 1 is a schematic, cross-sectional view of a gas turbine engine in accordance with an exemplary aspect of the present disclosure.
FIG. 2 is a close-up view of a combustion section of the exemplary gas turbine engine of FIG. 1 including an insertion tool in accordance with an exemplary embodiment of the present disclosure, along an axial direction and a radial direction.
FIG. 3 is another close-up view of the combustion section of the exemplary gas turbine engine of FIG. 1 including the exemplary insertion tool, along the radial direction and a circumferential direction.
FIG. 4 is a schematic view of a drive portion of the exemplary insertion tool of FIG. 3.
FIG. 5 is a side schematic view of an insertion portion of the exemplary insertion tool of FIG. 4.
FIG. 6 is a perspective view of a segment of an insertion tool in accordance with an exemplary embodiment of the present disclosure.
FIG. 7 is a cross-sectional view of a portion of the exemplary insertion tool of FIG. 5 in a bent position.
FIG. 8 is a side view of a portion of the exemplary insertion tool of FIG. 5 in a coupled position before adjustment.
FIG. 9 is a side view of a portion of the exemplary insertion tool of FIG. 5 in a coupled position after adjustment.
FIG. 10 illustrates a flow diagram of a method for adjusting the exemplary insertion tool in accordance with aspects of the present subject matter.

### DETAILED DESCRIPTION

Reference now will be made in detail to embodiments of the present disclosure, one or more examples of which are illustrated in the drawings. Each example is provided by way of explanation of the present disclosure, not limitation of the disclosure. In fact, it will be apparent to those skilled in the art that various modifications and variations can be made in the present disclosure without departing from the scope or spirit of the disclosure. For instance, features illustrated or described as part of one embodiment can be used with another embodiment to yield a still further embodiment. Thus, it is intended that the present disclosure covers such modifications and variations as come within the scope of the appended claims and their equivalents.

As used herein, the terms "first," "second," and "third" may be used interchangeably to distinguish one component from another and are not intended to signify location or importance of the individual components.

The terms "coupled," "fixed," "attached to," and the like refer to both direct coupling, fixing, or attaching, as well as indirect coupling, fixing, or attaching through one or more intermediate components or features, unless otherwise specified herein.

The singular forms "a," "an," and "the" include plural referents unless the context clearly dictates otherwise.

Approximating language, as used herein throughout the specification and claims, may be applied to modify any quantitative representation that could permissibly vary without resulting in a change in the basic function to which it is related. Accordingly, a value modified by a term or terms, such as "about," "approximately," "almost," and "substantially" are not to be limited to the precise value specified. In some instances, the approximating language may correspond to the precision of an instrument for measuring the value. In some instances, the approximating language may correspond to the precision of an instrument for measuring the value. For example, the approximating language may refer to being within a 1, 2, 4, 10, 15, or 20 percent margin. These approximating margins may apply to a single value, either or both endpoints defining numerical ranges, and/or the margin for ranges between endpoints. Here and throughout the specification and claims, range limitations are combined and interchanged, such ranges are identified and include all the sub-ranges contained therein unless context or language indicates otherwise. For example, all ranges disclosed herein are inclusive of the endpoints, and the endpoints are independently combinable with each other.

Finally, given the configuration of compressor and turbine about a central common axis, as well as the cylindrical configuration common to many combustor types, terms describing position relative to an axis may be used herein. In this regard, it will be appreciated that the term "radial" refers to movement or position perpendicular to an axis. Related to this, it may be required to describe relative distance from the central axis. In this case, for example, if a first component resides closer to the central axis than a second component, the first component will be described as being either "radially inward" or "inboard" of the second component. If, on the other hand, the first component resides further from the central axis than the second component, the first component will be described herein as being either "radially outward" or "outboard" of the second component. Additionally, as will be appreciated, the term "axial" refers to movement or position parallel to an axis. Finally, the term "circumferential" refers to movement or position around an axis. As mentioned, while these terms may be applied in relation to the common central axis that extends through the compressor and turbine sections of the engine, these terms also may be used in relation to other components or sub-systems of the engine.

At least certain gas turbine engines include, in serial flow arrangement, a compressor section including a low-pressure compressor and a high-pressure compressor for compressing air flowing through the engine, a combustor for mixing fuel with the compressed air such that the mixture may be ignited, and a turbine section including a high-pressure turbine and a low-pressure turbine for providing power to the compressor section.

Within one or more of the sections, at least certain gas turbine engines define an annular opening. Certain of these annular openings may vary in size, such that a dedicated, specialized insertion tool may be utilized with each annular opening to extend around and through such annular opening.

Accordingly, an improved tool for inspecting and performing maintenance within a gas turbine engine, and method of adjusting the improved tool during manufacture and/or assembly, would be welcomed in the art. In general, the present subject matter generally relates to a system and method for adjusting a tool for inserting into a cavity of a machine, such as a gas turbine machine. The tool may be used for inspecting an environment (e.g., inspecting internal components with the tool to produce two-dimensional images) and/or performing maintenance operations on the machine.

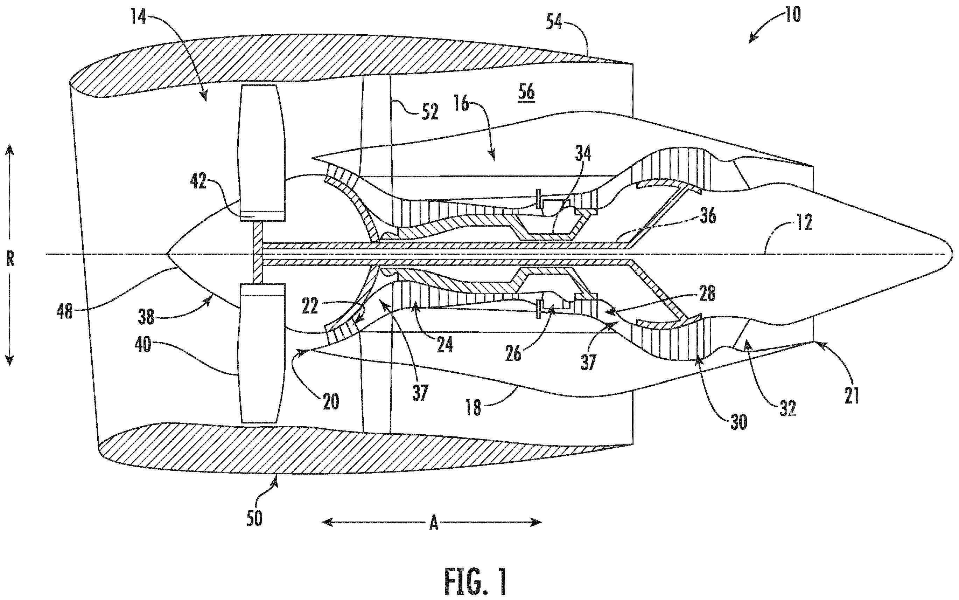

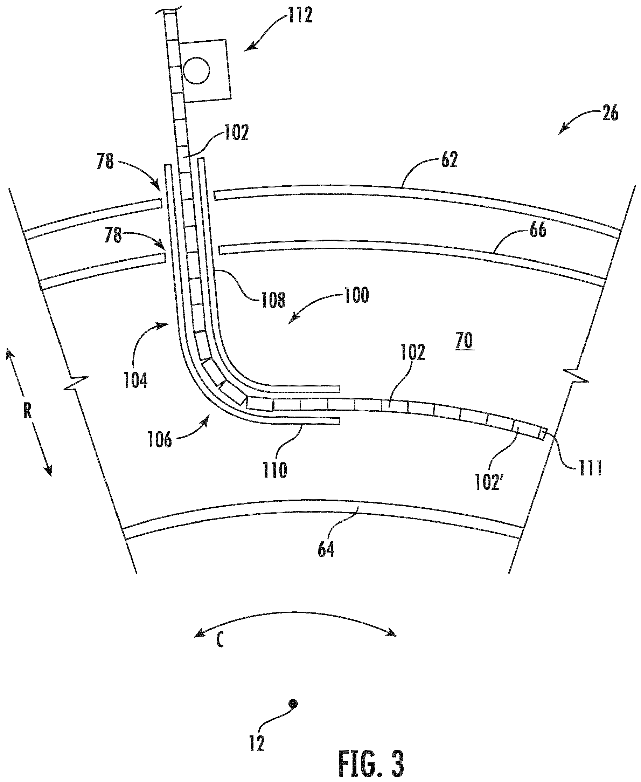

Referring now to the drawings, wherein identical numerals indicate the same elements throughout the figures, FIG. 1 is a schematic cross-sectional view of a gas turbine engine in accordance with an exemplary embodiment of the present disclosure. More particularly, for the embodiment of FIG. 1, the gas turbine engine is a high-bypass turbofan jet engine 10, referred to herein as "turbofan engine 10." As shown in FIG. 1, the turbofan engine 10 defines an axial direction A (extending parallel to a longitudinal centerline 12 provided for reference) and a radial direction R. The turbofan engine 10 also defines a circumferential direction C (see FIG. 3) extending circumferentially about the axial direction A. In general, the turbofan engine 10 includes a fan section 14 and a turbomachine 16 disposed downstream from the fan section 14.

The turbomachine 16 depicted is generally enclosed within an outer casing 18 that is substantially tubular and defines an annular inlet 20 and an annular exhaust 21. The outer casing 18 encases, in serial flow relationship, a compressor section including a booster or low-pressure (LP) compressor 22 and a high-pressure (HP) compressor 24; a combustion section 26; a turbine section including a high-pressure (HP) turbine 28 and a low-pressure (LP) turbine 30; and a jet exhaust nozzle section 32. A high-pressure (HP) shaft or spool 34 drivingly connects the HP turbine 28 to the HP compressor 24. A low-pressure (LP) shaft 36 or spool drivingly connects the LP turbine 30 to the LP compressor 22. The compressor section, combustion section 26, turbine section, and jet exhaust nozzle section 32 together define a core air flowpath 37 therethrough.

For the embodiment depicted, the fan section 14 includes a fixed pitch fan 38 having a plurality of fan blades 40. The fan blades 40 are each attached to a disk 42, with the fan blades 40 and disk 42 together rotatable about the longitudinal centerline 12 by the LP shaft 36. For the embodiment depicted, the turbofan engine 10 is a direct drive turbofan engine, such that the LP shaft 36 drives the fixed pitch fan 38 of the fan section 14 directly, without use of a reduction gearbox. However, in other exemplary embodiments of the present disclosure, the fixed pitch fan 38 may instead be a variable pitch fan, and the turbofan engine 10 may include a reduction gearbox, in which case the LP shaft 36 may drive the fixed pitch fan 38 of the fan section 14 across the gearbox.

Referring still to the exemplary embodiment of FIG. 1, the disk 42 is covered by rotatable front hub 48 aerodynamically contoured to promote an airflow through the plurality of fan blades 40. Additionally, the turbofan engine 10 includes an annular nacelle assembly 50 that circumferentially surrounds the fixed pitch fan 38 and/or at least a portion of the turbomachine 16. For the embodiment depicted, the annular nacelle assembly 50 is supported relative to the turbomachine 16 by a plurality of circumferentially-spaced outlet guide vanes 52. Moreover, a downstream section 54 of the annular nacelle assembly 50 extends over an outer portion of the outer casing 18 so as to define a bypass airflow passage 56 therebetween. The ratio between a first portion of air through the bypass airflow passage 56 and a second portion of air through the annular inlet 20 of the turbomachine 16, and through the core air flowpath 37, is commonly known as a bypass ratio.

It will be appreciated that although not depicted in FIG. 1, the turbofan engine 10 may further define a plurality of openings allowing for inspection of various components within the turbomachine 16. For example, the turbofan engine 10 may define a plurality of borescope openings at various axial positions within the compressor section, combustion section 26, and turbine section. Additionally, as will be discussed below, the turbofan engine 10 may include one or more igniter ports within, e.g., the combustion section 26 of the turbomachine 16, that may allow for inspection of the combustion section 26.

It should further be appreciated that the turbofan engine 10 depicted in FIG. 1 is by way of example only, and that in other exemplary embodiments, the turbofan engine 10 may have any other suitable configuration, including, for example, any other suitable number of shafts or spools, turbines, compressors, etc. The phrase "turbofan engine 10" may also refer to one or more components of the turbofan engine 10, e.g., a high-pressure compressor 24, a compressor section, and/or a combustion section 26. Further, in certain embodiments, "turbofan engine 10" may also refer to one or more modules. Additionally, or alternatively, in other exemplary embodiments, any other suitable turbine engine may be provided. For example, in other exemplary embodiments, the turbine engine may not be a turbofan engine, and instead may be configured as a turboshaft engine, a turboprop engine, turbojet engine, etc.

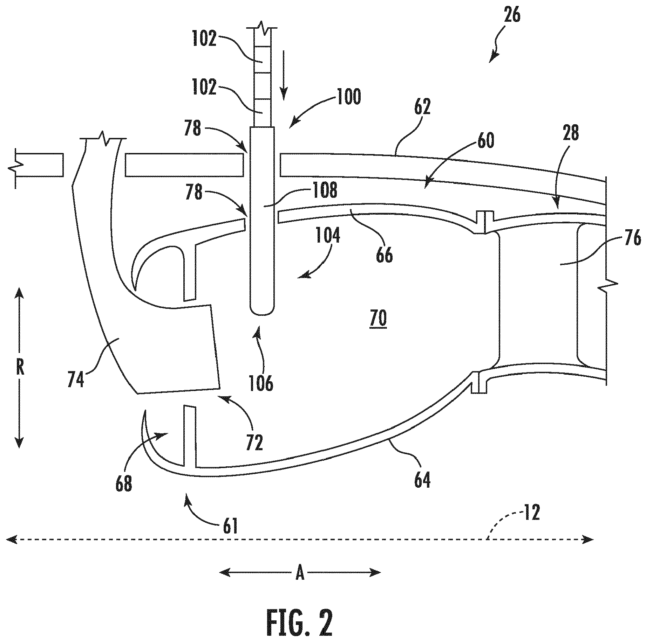

Referring now to FIG. 2, a close-up, cross-sectional view of the combustion section 26 of the turbomachine 16 of the turbofan engine 10 of FIG. 1 is provided along with a tool 100 for insertion into an annular section, of the turbofan engine 10. It will be appreciated that although the tool 100 is depicted in FIG. 2, and described below, as being inserted into a combustion section 26, in other exemplary embodiments, the tool 100 may additionally, or alternatively, be inserted into other cavities of the turbofan engine 10. For example, the tool 100 may be inserted into annular sections of the compressor section or the turbine section, or, alternatively, other engines, modules, and/or systems altogether. Additionally or alternatively, still, the tool 100 may be inserted into a non-annular section.

As is depicted, the combustion section 26 generally includes a combustor 60 positioned within a combustor casing 62. Additionally, the combustor 60 includes an inner liner 64, an outer liner 66, and a dome 68 together defining at least in part a combustion chamber 70. It will be appreciated that the dome 68, for the embodiment depicted, is an annular dome and the combustor 60 is configured as an annular combustor. In such a manner, the combustion chamber 70 generally defines an annular shape. At a forward end 61, the combustor 60 defines, or rather, the dome 68 defines, a nozzle opening 72, and the combustion section 26 further includes a fuel-air mixer 74, or nozzle, positioned within the nozzle opening 72. The fuel-air mixer 74 is configured to provide a mixture of fuel and compressed air to the combustion chamber 70 during operation of the turbofan engine 10 to generate combustion gases. The combustion gases flow from the combustion chamber 70 to the HP turbine 28, and more specifically, through a plurality of inlet guide vanes 76 of the HP turbine 28.

Notably, although a single nozzle opening 72 and fuel-air mixer 74 are depicted in FIG. 2, the combustor 60 may further include a plurality of circumferentially spaced nozzle openings 72 and a respective plurality of fuel-air mixers 74 positioned within the nozzle openings 72.

In order to initiate a combustion of the fuel and compressed air provided to the combustion chamber 70 by the fuel-air mixer 74, the combustion section 26 typically includes one or more igniters (not installed or depicted) extending through respective igniter openings 78 defined in the combustor casing 62 and the outer liner 66 of the combustor 60. However, when the turbofan engine 10 is not operating, the igniter(s) may be removed and the igniter opening(s) 78 may be utilized for inspecting, e.g., the combustion chamber 70, inlet guide vanes 76 of the HP turbine 28, and/or other components.

More specifically, for the embodiment of FIG. 2, the tool 100 capable of insertion into a cavity of an engine is depicted extending through the pair of igniter openings 78 defined in the combustor casing 62 and the outer liner 66 of the combustor 60. However, it will be appreciated that alternatively and/or additionally, the tool 100 may be inserted through one or more borescope ports and/or any other of the plurality of openings defined by the turbofan engine 10.

Referring now to FIG. 3, a partial, axial cross-sectional view of the combustion section 26 of FIG. 2 is shown. It will be appreciated that the tool 100 generally includes a plurality of segments 102 and an insertion tube 104, with the plurality of segments 102 movable through the insertion tube 104 into the combustion chamber 70.

Additionally, for the exemplary embodiment depicted, the insertion tube 104 includes a bend 106. For the embodiment shown, the bend 106 is a substantially 90-degree bend. For example, the insertion tube 104 includes a radial portion 108 extending substantially along the radial direction R and a circumferential portion 110 extending substantially along the circumferential direction C. The radial portion 108 and circumferential portion 110 are joined at the bend 106. In certain non-limiting embodiments, the radial portion 108 and the circumferential portion 110 comprise the same material such that the same material extends continuously between the radial portion 108 and the circumferential portion 110, thereby joining the two portions. The plurality of segments 102 are fed through the radial portion 108, pivot in a first angular direction relative to one another to go through the bend 106, and then pivot in a second, opposite angular direction relative to one another and couple to one another such that they are in a fixed position relative to one another as they move through to the circumferential portion 110. From the circumferential portion 110, the segments 102 extend through the combustion chamber 70.

Further, a forward-most segment 102' of the plurality of segments 102 can include an implement and/or motion axes. For example, as illustrated in Figure FIG. 3, the implement may comprise a camera 111 to allow the user to inspect various components of the combustor 60 and/or high-pressure turbine 28. It will be appreciated, however, that the tool 100 may include any other suitable implement, such that the tool 100 may be utilized for any suitable purpose. For example, the tool 100 may be utilized for various inspection activities. In particular, the tool 100 may be used to inspect the interior of the engine using, e.g., the camera 111. The tool 100 may alternatively or additionally include motion axes to move the camera 111 within the interior of the engine. For example, in some embodiments, the plurality of segments 102 may include windows in the sides of the plurality of segments 102 (not illustrated). The implement, e.g., a borescope, may be passed through the interior of the plurality of segments 102 once the tool 100 is deployed and may be operated through one or more windows in the sides of the plurality of segments 102. The implement may be used to position the tool 100 and also may be used to make adjustments to the plurality of segments 102, as described more in depth below. In still further embodiments, the implement may further include the camera 111, allowing the user to take one or more photographs of the tool 100 (e.g., through the windows in the sides of the plurality of segments 102) and making adjustments based on the one or more photographs.

Additionally, or alternatively, the tool 100 may include various other tool implements to perform one or more maintenance operations within the interior of the engine, e.g., drilling, welding, heating, cooling, cleaning, spraying, etc. In particular, the maintenance activities may include spraying thermal barrier coating shield materials, patch repairing thermal barrier coating, thermal spraying, removing coating (e.g., laser, waterjet, etc.), heat treating components (e.g., via flame, induction, and/or laser), and welding and brazing (e.g., laser, autogenous, and/or exothermic welding and brazing). In these embodiments, the tool 100 may likewise include motion axes to help move the tool 100 within and around the engine to conduct the one or more maintenance operations.

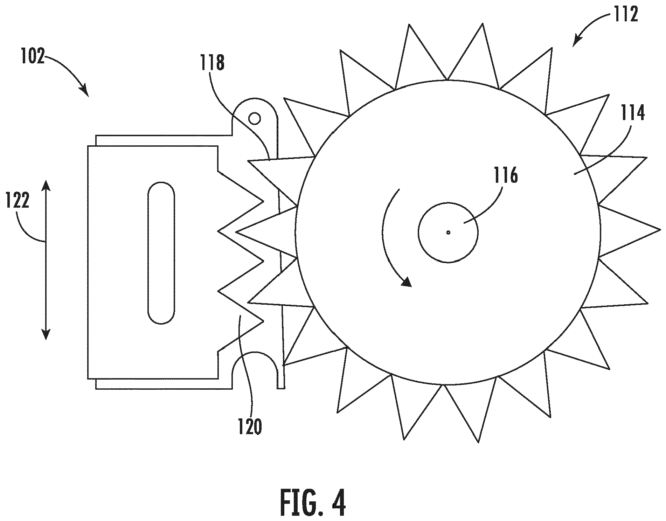

Further, the tool 100 includes a drive assembly 112 for driving the plurality of segments 102 of the tool 100 into, or out of, the interior of the engine, and more specifically for the embodiment shown, into or out of the combustion chamber 70, through the insertion tube 104. Referring now briefly to FIG. 4, providing a close-up, schematic view of the drive assembly 112 and a single segment 102A of the plurality of segments 102, it will be appreciated that the exemplary drive assembly 112 generally includes a drive wheel 114 and a drive motor 116. The drive wheel 114 includes a plurality of drive gear teeth 118 spaced along a circumference thereof, and the drive motor 116 is configured to rotate the drive wheel 114. For the embodiment shown, and as will be described in more detail below, each segment 102A of the plurality of segments 102 includes a drive feature, which for the embodiment shown, is a plurality of segment gear teeth 120. The plurality of segment gear teeth 120 of the segment 102A are each configured to mesh with the plurality of drive gear teeth 118 of the drive wheel 114, such that rotation of the drive wheel 114 by the drive motor 116 moves the segment 102A along a longitudinal direction 122 of the segment 102. Additionally, and/or alternatively, the drive feature of segment 102A may include a friction drive. Although not depicted, it will be appreciated that the drive motor 116 may be operably coupled to a controller or other control device, such that a length of the tool 100 within the interior of the engine may be controlled with relative precision by the drive assembly 112.

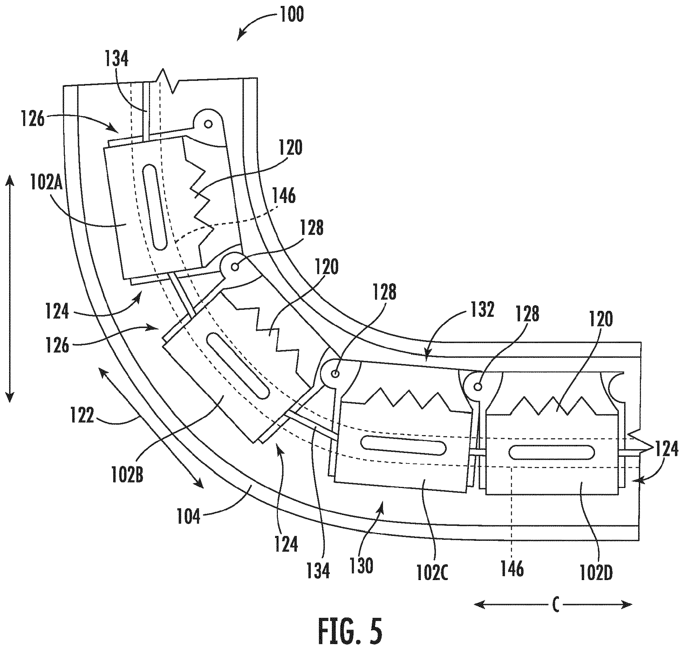

Referring now to FIG. 5, a close-up view of a portion of the tool 100 of FIGS. 2 and 3 is provided. Specifically, FIG. 5 provides a close-up view of four segments 102 of the plurality of segments 102 of the tool 100 extending through the bend 106 of the insertion tube 104. The plurality of segments 102 generally include a first segment 102A, a second segment 102B, a third segment 102C, and a fourth segment 102D.

Each of the plurality of segments 102 extend generally along a respective longitudinal direction 122 between a forward end 124 and an aft end 126, with the aft end 126 of the first segment 102A being pivotably coupled to the forward end 124 of a second segment 102B that is aft-adjacent to the first segment 102A, and the forward end 124 of the first segment 102A being pivotably coupled to the aft end 126 of a forward-adjacent segment, e.g., the third segment 102C. The third segment 102C may further be pivotably coupled to the fourth segment 102D, as shown in FIG. 5. As used herein, "pivotably coupled" refers to the first segment 102A being moveable relative to the second segment 102B between a bent position and a coupled position, where the first segment 102A is adjacent to the second segment 102B. Further, it will be appreciated, that as used herein, the term "longitudinal direction" with respect to a particular segment, e.g., first segment 102A, in the plurality of segments 102 refers to a direction extending between pivot axes 128 at the forward end 124 and aft end 126 of the first segment 102A where the first segment 102A is coupled to the second segment 102B and the third segment 102C, in a plane perpendicular to these pivot axes 128.

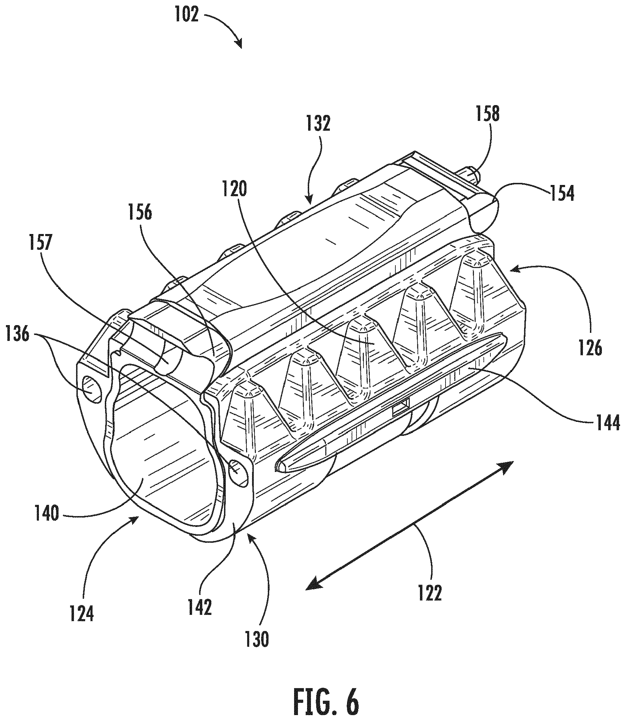

Notably, each of the first segment 102A, the second segment 102B, the third segment 102C, and the fourth segment 102D defines a respective outer side 132 and a respective inner side 130. The forward end 124 of the second segment 102B and the aft end 126 of the first segment 102A are pivotably coupled at their respective outer sides 132. Similarly, the forward end 124 of the first segment 102A and the aft end 126 of the third segment 102C are pivotably coupled at their respective outer sides 132, and the forward end 124 of the third segment 102C and the aft end 126 of the fourth segment 102D are pivotably coupled at their respective outer sides 132. It will be appreciated, however, that in other exemplary embodiments, the plurality of segments 102 may instead be pivotably coupled to one another at their respective inner sides 130, or a location between their respective outer side 132 and inner side 130. As is depicted in FIG. 6, the tool 100 additionally includes a biasing member, and more specifically a line assembly having one or more lines 134 configured to bias the segments 102 towards their respective coupled positions (discussed below).

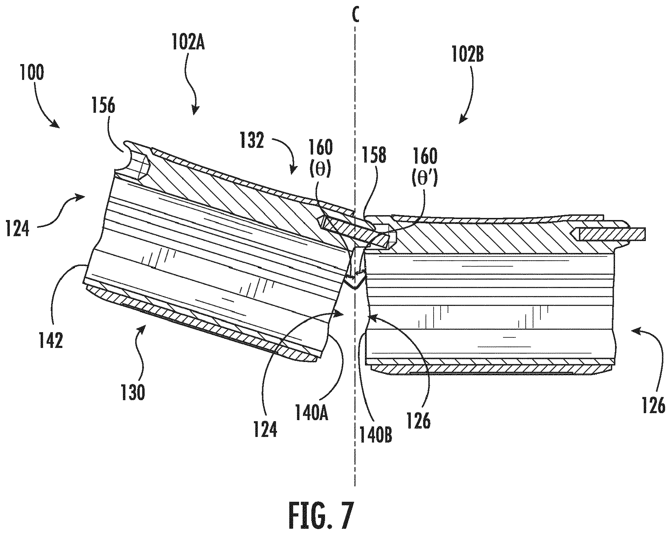

For the embodiment shown, and as will be explained in more detail below, the line 134 extends through the plurality of segments 102, and specifically, for the embodiment shown, through at least the first segment 102A, the second segment 102B, the third segment 102C, and the fourth segment 102D. As stated, the line 134 is configured to bias the plurality of segments 102 towards their respective coupled positions (discussed below), for example, to bias the first segment 102A towards the coupled position relative to the second segment 102B. For the embodiment shown, the line 134 is configured to extend through line guides 136 (see FIG. 7, discussed below) within each of the first segment 102A, the second segment 102B, the third segment 102C, and the fourth segment 102D for providing a biasing force to press the first segment 102A, the second segment 102B, the third segment 102C, and the fourth segment 102D together.

In certain exemplary embodiments, the line 134 may be configured as a metal line, or any other suitable material or line. However, in still other embodiments, any other suitable biasing member may be provided. For example, in some embodiments, the line 134 may be a plurality of lines, with each line 134 in the plurality of lines extending between a pair of adjacent segments 102 of the tool 100, or, alternatively, with each line 134 extending from a base of the tool 100 to an individual segment, e.g., the first segment 102A, to provide the biasing of the first segment 102A towards a coupled position relative to an aft-adjacent segment 102. Additionally or alternatively, the biasing member may be a plurality of springs extending between adjacent segments in the plurality of segments 102, with each spring oriented axially to pull the plurality of segments 102 together or oriented torsionally to bendably bias the plurality of segments 102 towards each other by rotation about their respective pivot axis 128. The biasing member may further bias the adjacent segments in the plurality of segments 102 into a J-tube or other similar structure for adjustments (as described more in depth below), and/or to bias the adjacent segments in the plurality of segments 102 for insertion into the machine when the tool 100 is deployed. Further, in still other exemplary embodiments, the biasing member may not be a tension member, and instead may be any other suitable biasing member, such as one or more magnets and/or ferromagnetic materials. Additionally, and/or alternatively, the biasing member may comprise shape memory alloy, which may be used by itself and/or in conjunction with any of the other described biasing members. For example, shape memory alloy may be used with ferromagnetic materials and/or heat phase changes to control the amount of biasing. In an exemplary embodiment, the biasing member comprising shape memory alloy may be flexible and bias accordingly when entering the J-tube. The biasing member with shape memory alloy may further be able to rigidize, e.g., return to its initial shape, after passing through the J-tube. In still other embodiments, the biasing member may further include actuators to control the biasing member.

FIG. 6 provides a perspective view of the first segment 102A of a plurality of segments 102 of the tool 100, and FIG. 7 provides a side view of the first segment 102A and the second segment 102B of the tool 100. As will be appreciated from the views of the segment 102A and the second segment 102B depicted in FIGS. 6 and 7, each first segment 102A of the plurality of segments 102 can comprise at least two different materials specifically designed to impart particular mechanical properties to each first segment 102A of the plurality of segments 102.

Additionally, the core 140 may be formed through one or more traditional manufacturing methods, such as by casting, machining, laser cutting and bending, extruding, 3D printing/additive manufacturing, etc. The geometry of the core 140 allows it to be formed with a relatively hard and stiff material through a variety of manufacturing methods.

By contrast, the shell 142 of the first segment 102A may be overmolded onto the core 140 subsequent to the formation of the core 140. For the embodiment shown, at least part of the shell 142 covers at least part of the exterior of the core 140. For example, in only certain exemplary embodiments, the shell 142 may cover at least about 10% of the surface area of the exterior of the core 140, at least about 25% of the exterior of the core 140, at least about 50% of the exterior of the core 140, at least about 60% of the exterior of the core 140, at least about 75 % of the exterior of the core 140, or at least about 90% of the exterior of the core 140 (with the "exterior" of the core 140 being the portion of the core 140 that would otherwise be viewable when the plurality of segments 102 are in the coupled position). In such a manner, it will be appreciated that the shell 142 of the first segment 102A may be formed through a thermal melt-based molding process. However, in other embodiments, the shell 142 of the first segment 102A may be formed through any other suitable process, such as through a reaction injection molding process, which may more easily allow for molding of materials which are not plastics.

As will be explained in more detail below, the shell 142 may include more complex geometries than the core 140, such that it may be relatively easy to form the shell 142 with a more moldable material, e.g., one or more of the materials listed above as exemplary second materials. Further, forming the first segment 102A in such a manner may allow for features included with or defined by the shell 142 of the first segment 102A to have different mechanical properties than the core 140. However, it will be appreciated that the complex geometries may alternatively be formed using the first material and/or included in the core 140. Additionally, and/or alternatively, the core 140 may be bonded to the shell 142, e.g., through adhesive bonding and/or any other method of joining two plastics.

As noted above, the shell 142 may include or define more complex geometries than the core 140. Specifically, in the embodiment shown in FIG. 6, the first segment 102A includes or defines a guide feature, a drive feature, a line guide 136, or a combination thereof. More specifically, for the embodiment shown, the shell 142 includes or defines each of the guide feature, the drive feature, and the line guide 136.
For example, the drive feature of the shell 142 includes the plurality of gear teeth 120, which are configured to mesh with the plurality of drive gear teeth 118 of the drive assembly 112 described above with reference to FIG. 5. In such a manner, the plurality of segment gear teeth 120 to be configured to move the first segment 102A forward or back during operation. It will be appreciated, however, that in other embodiments, the drive feature of the shell 142 may be configured in any other suitable manner. For example, in other embodiments, the drive feature may be one or more differently configured drive gear teeth 118, or alternatively may be any other suitable geometry for providing friction for a drive assembly 112, such as the exemplary drive wheel 114, to grip the first segment 102A and move first segment 102A forward or back. For example, the drive feature may be a plurality of ridges, or other structure.

Referring to FIG. 6 again, for example, the first segment 102A can include a core 140 comprising a first material and a shell 142 comprising a second material. It will be understood that in one example the first material is different from the second material. For example, the first material defines a first material stiffness and the second material defines a second material stiffness. In some embodiments, the stiffness of the first material can be greater than the stiffness of the second material. For example, in some embodiments, the first material stiffness is at least about five (5) times greater than the second material stiffness as measured in a suitable engineering unit for stiffness, such as gigapascals (GPa). In some embodiments, the first material stiffness may be at least about 20 times greater than the second material stiffness, including but not limited to that the first material stiffness may be at least about 50 times greater than the second material stiffness.

In some embodiments, the first material, forming the core 140 of the first segment 102A, may be a relatively stiff material defining a stiffness greater than about 100 GPa, such as greater than about 125 GPa, such as greater than about 175 GPa, such as up to about 12,000 GPa. By contrast, the second material, forming the shell 142 of the first segment 102A, maybe a relatively low stiffness material defining a stiffness less than about 100 GPa, such as less than about 75 GPa, such as less than about 50 GPa, such as less than about 25 GPa, such as at least about 0.01 GPa. By way of non-limiting examples, the first material may comprise one or more of a metal material (such as a titanium or titanium alloy, copper, aluminum or aluminum alloy, magnesium or magnesium alloy, steel, or stainless steel), may comprise a ceramic material (such as a reinforced ceramic, such as a whisker reinforced ceramic or other fiber reinforced ceramic), or may comprise a carbon fiber reinforced plastic, a glass fiber reinforced plastic, or a lubrication-infused fiber reinforced plastic. Also, by way of non-limiting examples, the second material may comprise one or more of a polymer, a plastic polymer (such as an acetal polymer, acrylonitrile butadiene styrene (ABS), polycarbonate (PC), polyoxymethylene (POM), polystyrene, or polyvinyl chloride (PVC)), or may comprise a rubber material.

Utilizing a different second material for the shell 142 and the first material for the core 140 may allow for the shell 142 to have different mechanical properties than the core 140. For example, as discussed above, it may be important for the core 140 to have a relatively high stiffness, such that the tool 100 defines a relatively high overall stiffness during operation. However, such may not necessary be a mechanical property that is important or desirable for shell 142. In particular, for the embodiment discussed herein with respect to FIGS. 6 and 7, it may be desirable for the drive feature of the shell 142 to be configured to wear more quickly than, e.g., the drive gear teeth 118 of the drive wheel 114. For example, it will be appreciated that the second material defines a second material hardness and the drive gear teeth 118 of the drive wheel 114 may comprise a material defining a material hardness greater than the second material hardness. In such manner, the drive feature of the shell 142 may be configured to wear down more quickly than the drive gear teeth 118 of the drive wheel 114, which may be desirable given that each drive gear tooth 118 is likely to engage with segment gear teeth 120 many more times than each segment gear tooth 120 is likely to engage with drive gear teeth 118.

In addition, the shell 142, comprising the second material, includes the guide feature. For the embodiment shown, the guide feature is a segment sliding plain bearing feature 144. The segment sliding plain bearing feature 144 may be configured to guide the first segment 102A through the insertion tube 104, while also ensuring the first segment 102A maintains a desired orientation within the insertion tube 104. It will be appreciated that the insertion tube 104 similarly includes a tube sliding plain bearing feature 146 (not shown). The tube sliding plain bearing feature 146 is configured to interact with the segment sliding plain bearing feature 144. In particular, the tube sliding plain bearing feature 146 is configured as a channel or other indentation in a wall of the insertion tube 104, and the segment sliding plain bearing feature 144 is configured as a linear protrusion extending outward from the core 140 (and proud of the surrounding portions of the shell 142) and along the longitudinal direction 122 of the first segment 102A. In such a manner, the segment sliding plain bearing feature 144 may be positioned at least partially within the tube sliding plain bearing feature 146 to prevent the first segment 102A from becoming misaligned or twisted out of orientation when being inserted through the insertion tube 104.

Notably, by forming the shell 142 of the second material, separate from the first material of the core 140, the shell 142 may comprise material to facilitate the segment sliding plain bearing feature 144 operating as desired with, e.g., the tube sliding plain bearing feature 146. For example, it will be appreciated that in only certain exemplary embodiments, such as the exemplary embodiment depicted, the first material may define a first coefficient of friction and the second material may define a second coefficient of friction. The first coefficient of friction is at least about fifteen percent greater than the second coefficient of friction (i.e., µ(first material) = µ(second material) x 1.15). Specifically, in at least some embodiments, the first coefficient of friction may be at least about thirty percent greater than the second coefficient of friction, such as at least about fifty percent greater than the second coefficient of friction, such as up to about 1,000% greater than the second coefficient of friction. Such a configuration may enable the first segment 102A to relatively easily slide along within the insertion tube 104, without necessitating, e.g., lubricated bearings or other more complex mechanical structures, lubrications, etc.

Further, as with the drive feature, it may be beneficial for the segment sliding plain bearing feature 144 to wear more quickly than the tube sliding plain bearing feature 146 of the insertion tube 104. As such, it will be appreciated that in at least certain exemplary embodiments, a material hardness of the material forming or defining the tube sliding plain bearing feature 146 may be greater than the second material hardness of the second material forming the shell 142 and the segment sliding plain bearing feature 144.

It will be appreciated, however, that although the exemplary illustration of the tool 100 depicted includes sliding plain bearing features 144, 146, in other exemplary embodiments, other guide features may be provided. For example, in other embodiments, the shell 142 of the segments 102 may incorporate a roller bearing design, or the tube sliding plain bearing feature 146 may additionally or alternatively utilize a roller bearing design. Additionally, or alternatively, still, one or both of the sliding plain bearing features 144, 146 may be replaced with or supplemented with, e.g., air bearing features, lubrication bearing features, etc.

Moreover, as noted above, the shell 142 includes the line guide 136. For the embodiment shown, the line guide 136 extends substantially from the forward end 124 of the first segment 102A to the aft end 126 of the first segment 102A along the longitudinal direction 122 the first segment 102A. As discussed above, the second material may define a relatively low coefficient of friction. Such may assist with threading the line 134 through the line guides 136 of the various segments 102. As also discussed above, the second material may define a relatively low material hardness. In certain exemplary embodiments, the line 134 may comprise a line material defining a line material hardness greater than the second material hardness. Such may ensure that operation of the tool 100 does not appreciably wear down the line 134, such wear potentially resulting in a failure of the tool 100 within an environment.

Referring to FIGS. 6 and 7, it will be appreciated that adjacent segments 102 are pivotably contacting one another at a joint 152, which for the embodiment shown is positioned generally at the outer side 132. The joint 152 is formed, for the embodiment shown, of a pair of rounded protrusions 154 on a first segment 102A and a corresponding pair of indention members 156 on a second segment 102B (see FIG. 7). The rounded protrusions 154 and indention members 156 have corresponding geometries, and each of the pair of rounded protrusions 154 and indention members 156 are spaced from one another in a cross-wise direction along the pivot axis 128. The rounded protrusions 154 and indention members 156 are, for the embodiment shown, formed as part of the core 140. However, the segments 102 shown further include an alignment feature 158 positioned between one of the pair of rounded protrusions 154 or pair of indention members 156 for extending into an opening 157 between the other of the pair of rounded protrusions 154 or indention members 156. Particularly for the embodiment shown the alignment feature 158 is positioned between the pair of rounded protrusions 154 and extends between the pair of indention members 156 to assist with aligning the adjacent segments 102. For the embodiment shown, the alignment feature 158 is formed as part of the shell 142. However, it will be appreciated that the alignment feature 158 may alternatively be formed separately and then coupled to the shell 142.

Briefly, referring still to FIG. 7, it will be appreciated that the plurality of segments 102 may define an interior opening 148 allowing for supporting structure for the various tool implements described above. In particular, for the embodiment shown, the tool 100 includes a variety of structures 150 extending along a length of the plurality of structures encasing, e.g., fluid flow paths, electrical lines, etc. In such a manner, the opening through the plurality of segments 102 may enable operation of a wide variety of tool implements at, e.g., a distal end of the plurality of segments 102, such as at the forward-most segment 102'.

In view of the above description, it will be appreciated that forming the shell 142 of the second material may allow for the first segment 102A to maintain a desired stiffness and strength, while also having a variety of relatively complex geometry features, with these relatively complex geometry features defining material properties having specific benefits that would otherwise be difficult to obtain. Moreover, forming the shell 142 of the second material may reduce the weight of the first segment 102A, and therefore, a weight of the tool 100.

Furthermore, in some embodiments, the first material and/or the second material may be adjustable. In other words, the adjustable material may be adjusted to change one or more of its dimensional measurements. As used herein, "adjustable," "adjust," "adjusted," and variations thereof refers to changing one or more dimensional measurements of a material such as by removing material (e.g., abrading, etching, eroding, stripping, corroding, grinding, electrical discharge machining (EDM), electrolysis, cutting, dissolving, ablating, filing, etc.), adding material (e.g., welding, printing, sintering, soldering, electrolytic plating, plasma spraying, epoxy additives, coating, painting, physical vapor deposition (PVD), depositing, etc.), or otherwise altering a physical dimension of the originally provided material. In other words, adjusting at least one of the first segment 102A and the second segment 102B may include removing material from and/or adding material to at least one of the first segment 102A and the second segment 102B. It will be appreciated that adjustment may be achieved by any means as known to those skilled in the art. For example, the adjustment may include mechanical means, electrical means, chemical means, or any combination thereof. Moreover, in some embodiments, adjustment may be realized by first adding material and then removing material, or vice versa. It will also be appreciated that adjusting the tool 100 may additionally or alternatively include adjusting the tool 100 as a whole, rather than on a segment-by-segment basis, as discussed more in depth below.

In some embodiments, the second material can be adjusted and the first material cannot be adjusted (such as when the first material comprises a very high hardness and/or toughness). In other embodiments, the first material can be adjusted and the second material cannot be adjusted. In yet other embodiments, both the first material and the second material can be adjusted.

In still further embodiments, the first segment 102A may further comprise a third material, which may be the adjustable material. According to some embodiments, the third material is different from both the first material and the second material. The third material may be layered on top of the first material of the core 140 and/or on top of the second material of the shell 142. In these embodiments, the third material layer may be relatively thin, and third material may be stiffer, e.g., more brittle, than the first material of the core 140 and/or the second material of the shell 142. In other embodiments, the third material may help enable adjustments to the first material and/or the second material, such as by making the first material and/or the second material more easily adjustable and/or more brittle.

The third material may alternatively be the same as an adjusting material 162, where the adjusting material 162 is a material that is used to adjust the adjustable material. For example, if the adjustable material is an abradable material, e.g., a material that can be adjusted by abrasion, the adjusting material 162 may likewise be an abradable material such that rubbing the abradable material against the adjusting material 162 abrades both materials. It will be appreciated that the term "abradable" as used herein refers to a material that is capable of being abraded, ground, or otherwise mechanically eroded. It will be appreciated that an abrasive adjusting material 162 may comprise the materials mentioned above but may also comprise other abradable materials that are outside of the scope of examples provided. Similarly, the terms "abrasive" as used herein refers to material that is capable of grounding or otherwise eroding.

Referring now to FIG. 5, in some embodiments, the core 140 of one segment (e.g., third segment 102C) is configured to abut a core of an aft-adjacent segment (e.g., fourth segment 102D) in the coupled position. Additionally, although not shown, the core 140 of one segment (e.g., a first segment 102A) may be configured to abut a core of a forward adjacent segment (e.g., third segment 102C) and a core of an aft-adjacent segment (e.g., second segment 102B) in the coupled position. After the first segment 102A is formed or otherwise provided, the tool 100 can be at least partially assembled and adjusted. As used herein, "partially assembled" and "partial assembly" (and variants thereof) of the tool 100 means the first segment 102A is coupled to at least one other segment, e.g., second segment 102B.

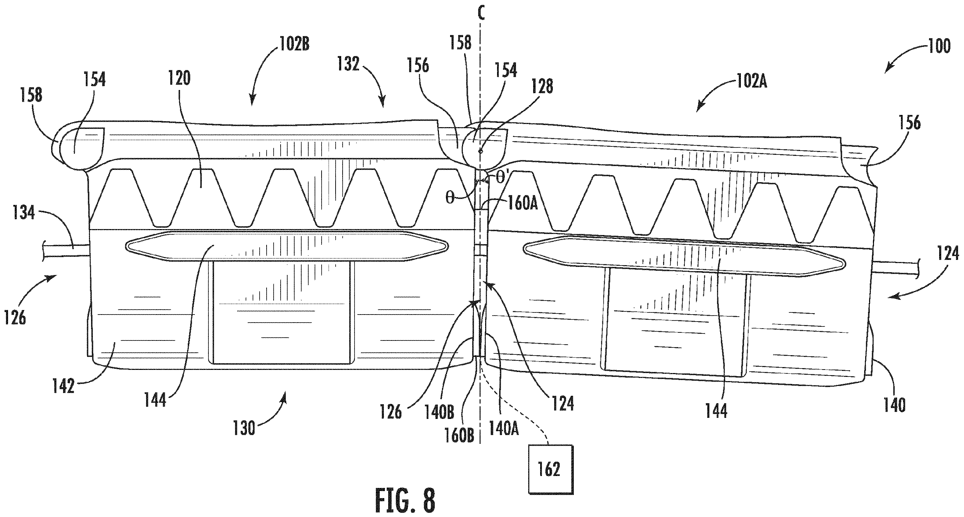

Referring now to FIGS. 7 and 8, at least a portion of the tool 100 defines an initial alignment measurement 160, where the initial alignment measurement 160 is shown with respect to the first segment 102A and the second segment 102B in the bent and coupled positions, respectively. As used herein, the term "initial alignment measurement" refers to a dimensional measurement relating to at least one segment before any adjustments are made. In FIGS. 7 and 8, the initial alignment measurement 160 is shown as an angle θ or θ', as defined from a centerline axis C, between the first segment 102A and the second segment 102B in the bent and coupled positions, respectively. The initial alignment measurement 160 (e.g., θ and θ') may be taken for either or both of the first segment 102A and the second segment 102B, as shown in the figures. Additionally or alternatively, in other embodiments, the initial alignment measurement 160 may refer to a thickness or a length of at least one of the core 140 and shell 142 in the coupled and/or bent position. Additionally, it will be appreciated that the initial alignment measurement 160 may refer to any quantifiable measurement or measurements between the first segment 102A and the second segment 102B in the coupled and/or bent position.

In some embodiments, this initial alignment measurement 160 is taken after the plurality of segments 102 has been assembled into the tool 100 and/or after the tool 100 has been deployed within the machine. In some embodiments, the shape of the tool 100 as a whole is measured as the initial alignment measurement 160 before adjustments are made to the tool 100, as explained more in depth below.

After the initial alignment measurement 160 is determined, measured, or otherwise sensed, it will be understood that the tool 100 may be at least partially adjusted until a target alignment measurement 165 is reached. The term "target alignment measurement" as used herein refers to the desired measurement relating to at least one segment of the plurality of segments 102. It will be understood that the target alignment measurement 165 is related to the initial alignment measurement 160 (e.g., is measuring the same dimensional parameter and/or has the same unit of measurement). Accordingly, if the initial alignment measurement 160 refers to an angle, then the target alignment measurement 165 also refers to an angle. If the initial alignment measurement 160 refers to a distance, then the target alignment measurement 165 also refers to a distance.

Further, the adjustment may be made to at least a portion of the plurality of segments 102, such as the first segment 102A, the second segment 102B, the third segment 102C, and/or the fourth segment 102D. It will also be appreciated that the adjustment may be made to the tool 100 as a whole.
In exemplary embodiments, the initial alignment measurement 160 and the target alignment measurement 165 are different; accordingly, the initial alignment measurement 160 and the target alignment measurement 165 are used to determine the adjustment amount. The term "adjustment amount" as used herein generally refers to the difference between the target alignment measurement 165 and the initial alignment measurement 160. In this embodiment, the adjustment amount would be correlated to the amount of material needed to be removed from, added to, or both, from the first segment 102A to achieve the target alignment measurement 165.

In one specific non-limiting embodiment where the initial alignment measurement 160 refers to an initial thickness of the core 140 (e.g., the thickness after the core 140 is manufactured) and the target alignment measurement 165 refers to the desired thickness of the core 140 that is less than the initial thickness of the core 140, the adjustment amount would be the amount of material of the core 140 to be removed during the adjustment process. Similarly, if the adjustment amount is of a negative value, e.g., where the desired thickness of the core 140 is greater than the initial thickness of the core 140, then the material of the core 140 can be added to during the adjustment.

In non-limiting embodiments, at least one of the first material and the second material of the first segment 102A can be partially removed and/or added to in order to adjust the fit tolerance with an adjacent segment, e.g., the second segment 102B, when the first segment 102A and the second segment 102B are in the coupled or bent position.

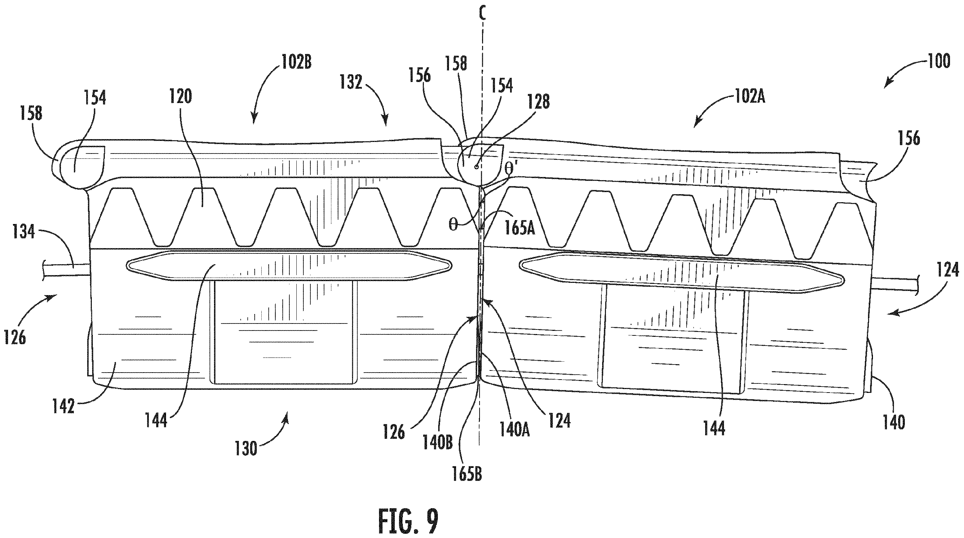

Referring to FIGS. 8 and 9 collectively, FIG. 8 shows the first segment 102A and the second segment 102B before the initial alignment measurement 160 is taken. FIG. 9 shows the first segment 102A and the second segment 102B after the adjustment has been made, e.g., where the target alignment measurement 165 has been achieved. As shown in FIG. 8, the initial alignment measurement 160 may refer to a length of the distance between the core 140 of the first segment 102A and the core 140 of the second segment 102B. In certain non-limiting embodiments, the initial alignment measurement 160 may include two or more measurements. For example, a first initial alignment measurement 160A may be taken across the alignment feature 158, e.g., a hinge, between the first segment 102A and the second segment 102B. Further, a second initial alignment measurement 160B may be taken between the first segment 102A and the second segment 102B along an edge opposite the alignment feature 148, e.g., where a first core 140A of the first segment 102A touches a second core 140B of the second segment 102B. In this exemplary embodiment, the adjustment may be made to either the first core 140A or the second core 140B. FIG. 8 further shows the adjusting material 162 being placed between the first segment 102A and the second segment 102B. In the depicted embodiment, the adjusting material 162 is removing the first material from the first core 140A and/or the second core 140B.

Referring now to FIG. 9, the first segment 102A and the second segment 102B are shown after the adjustment, e.g., where the target alignment measurement 165 has been reached. As shown, the distance between the first segment 102A and the second segment 102B is smaller and the first segment 102A and the second segment 102B are closer together.

However, it will be appreciated that the initial alignment measurement 160 may be substantially close enough to the target alignment measurement 165 such that an adjustment by removing and/or adding material may not be necessary. The tool 100 may instead be adjusted in other ways, as described more in depth below.

Additionally, the plurality of segments 102, when in the coupled position, may define a desired overall stiffness for the tool 100. More specifically, by having the core 140 of the first segment 102A abut the cores 140 of the adjacent segments, e.g., a second core of the second segment 102B and a third core of the third segment 102C, the plurality of segments 102 in the coupled position may together define the desired overall stiffness for the tool 100, allowing the tool 100 to extend a desired length within the environment, while still being capable of moving with a desired precision and/or carry a desired load. Furthermore, in a particular embodiment, the plurality of segments 102 are loaded to a representative working load prior to determining the initial alignment measurement 160. The representative working load and shape of the tool 100 may depend on a path of the tool 100, as described more in depth below.

In some embodiments, the adjustment amount may be the same for all segments of the plurality of segments 102 of the tool 100. This would allow the adjustment method 200, discussed more in depth below, to be streamlined and would allow for machine implementation, thereby increasing efficiency of the adjustment and assembly processes of the tool 100. However, in other embodiments, the adjustment amount may vary from the first segment 102A to the second segment 102B. In other words, one or more of the plurality of segments 102 may comprise a unique dimensional measurement affecting a fit tolerance, such as with an adjacent segment (e.g., the second segment 102B), when in the coupled position. This may lead to portions of the tool 100 being more non-uniform, allowing for greater flexibility and/or sag adjustment in the specific configuration of the tool 100. For example, the tool 100 may include a tip at one end of the tool 100, thereby allowing the user to define the path of the tool 100 by directing with the tip of the tool 100. The rest of the tool 100 accordingly follows the path defined by the tip of the tool 100. The tool 100 having portions that are non-uniform may additionally allow for varying representative working loads at different deployment amounts and help adjust for sag. Furthermore, the flexible configuration of the tool 100 may also allow the user to adjust the tool 100 to an intentionally unique path, e.g., a non-circular path. In other embodiments, the tool 100 may lie on a circle in one position, such as when the tool 100 is under the influence of gravity in a defined orientation.

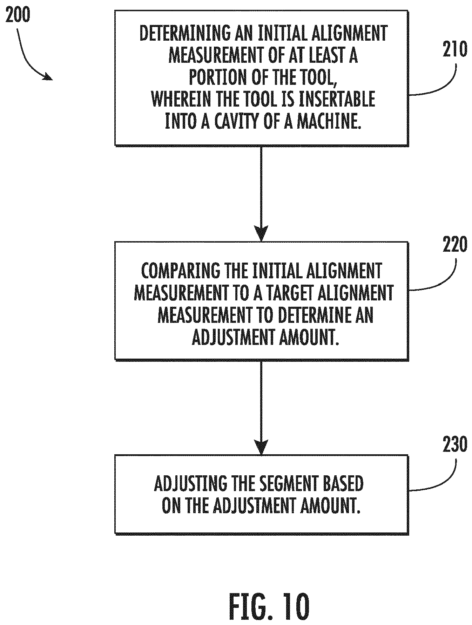

Referring now to FIG. 10, a flowchart of an exemplary method 200 is illustrated for adjusting the tool 100 comprising a plurality of segments 102. The exemplary method 200 includes, at 210, determining an initial alignment measurement 160 of at least a portion of the tool 100, where the tool 100 is insertable into a cavity of a machine; at 220, comparing the initial alignment measurement 160 to a target alignment measurement 165 to determine an adjustment amount; and, at 230, adjusting at least one of the plurality of segments 102 based on the adjustment amount.

At 210, the initial alignment measurement 160 of at least a portion of the tool 100 is determined. As mentioned above, the initial alignment measurement 160 is related to the dimensions of one or more segments of the plurality of segments 102. In some embodiments, a first segment 102A of the plurality of segments 102 is adjacent to a second segment 102B of the plurality of segments 102, wherein the first segment 102A and the second segment 102B are moveable between a bent position and a coupled position. Determining the initial alignment measurement 160 of at least a portion of the tool 100 may include determining the initial alignment measurement 160 of the first segment 102A and the second segment 102B in the coupled position. In certain embodiments, adding the first segment 102A to the second segment 102B may be included in the method 200, where adding the first segment 102A to the second segment 102B occurs prior to determining the initial alignment measurement 160 of the tool 100.

In particular, the initial alignment measurement 160 may refer to a measurement corresponding to the contacting face of the core 140, e.g., a first core 140A of the first segment 102A and/or a second core 140B of the second segment 102B. Moreover, in the exemplary embodiment, the initial alignment measurement 160 is an initial angle θ, θ' and/or distance between the first segment 102A and the second segment 102B in the coupled position. The first segment 102A and the second segment 102B may also and/or alternatively be fixed to each other. Additionally, in certain non-limiting embodiments, there may be 6 degrees of freedom between the first segment 102A and the second segment 102B. The initial alignment measurement 160, the target alignment measurement 165, and/or the adjustment may be in any dimension and/or any combination of dimensions of the 6 degrees of freedom.

At 220, the initial alignment measurement 160 is compared to a target alignment measurement 165 to determine an adjustment amount. As mentioned previously, the target alignment measurement 165 is the desired measurement relating to at least one segment 102. The adjustment amount is determined by calculating the difference between the target alignment measurement 165 and the initial alignment measurement 160, e.g., by subtracting the initial alignment measurement 160 from the target alignment measurement 165. In one particular embodiment, e.g., where the initial alignment measurement 160 measures the angle θ between the first segment 102A and the second segment 102B, the target alignment measurement 165 is a target angle θT between the first segment 102A and the second segment 102B. As stated previously, the adjustment amount can be made in any dimension and/or combination of dimensions of the 6 degrees of freedom.

At 230, the method includes adjusting at least one of the plurality of segments 102 based on the adjustment amount. It will be appreciated that, as described above, the adjustment amount is the difference between the target alignment measurement 165 and the initial alignment measurement 160. For example, where the adjustment amount is a negative value, e.g., where the target alignment measurement 165 is less than the initial alignment measurement 160, the method 200 could be applied to add material to the segment 102. In this embodiment, the adjusting material 162 may be a material that is capable of depositing material onto the segment 102. For example, adjusting the first segment 102A may include building up the first material, e.g., the core 140 of the segment 102. The adjusting material 162 may additionally, or alternatively, build up the surface of the second material, e.g., the shell 142 of the first segment 102A. Alternatively, where the adjustment amount is a positive value, e.g., where the target alignment measurement 165 is greater than the initial alignment measurement 160, the adjusting material 162 may remove material from the first segment 102A. Further, in some embodiments, removing and/or adding material from and/or to the first segment 102A based on the adjustment amount may include removing and/or adding material from and/or to the first segment 102A, the second segment 102B, or both.

For example, the adjustments may include one or more of directed energy deposition (DED), friction or ultrasonic welding, adding epoxy, dabber TiG welding, grinding, using emery paper, soldering, plasma spraying, physical vapor deposition (PVD), as well as any other methods mentioned herein. In certain embodiments, the first segment 102A is adjusted to the target alignment measurement 165 using the adjusting material 162 which removes and/or adds the adjustment amount from the first segment 102A. For example, the adjusting material 162 may be an abrasive material and the adjustable material may be an abradable material, such as sandpaper and/or emery paper, grains or ridges on a grinding tool, or any other material capable of mechanically removing abradable material. In other embodiments, the adjusting material 162 may be a chemical material and/or a tool may chemically dispose or remove the adjusting material 162 onto or from the first segment 102A.

Additionally, in certain embodiments where the initial alignment measurement 160 is taken between the first segment 102A and the second segment 102B in the coupled position, adjusting the first segment 102A based on the adjustment amount may further include adjusting at least the first segment 102A when the first segment 102A and the second segment 102B are in the bent position. The bent position configuration may allow for easier adjustments. For example, the first segment 102A and the second segment 102B may be placed in the bent position after the initial alignment measurement 160 is taken so that the adjusting material 162 may be inserted between the first segment 102A and the second segment 102B. In some embodiments, the first segment 102A and the second segment 102B may then be reconfigured in the coupled position so that the adjusting material 162 may remove and/or deposit material on one or both of the first segment 102A and the second segment 102B. Alternatively, the first segment 102A and the second segment 102B may remain in the bent position when the adjustments are made.

It will be appreciated that adjusting at least one of the plurality of segments 102 based on the adjustment amount may further include making adjustments in accordance with other methods described more in depth below.

In some embodiments, the method 200 may further include holding the first segment 102A (and/or any other segment in the plurality of segments 102) in a fixed position prior to adjusting the first segment 102A based on the adjustment amount. Specifically, holding the first segment 102A in the fixed position may include using one or more jig assemblies.

Additionally, the method 200 may be applied to more than just two adjacent segments such as the first segment 102A and the second segment 102B. For example, the first segment 102A and the second segment 102B may define a first set of two adjacent segments with a first initial alignment measurement 160A and the method 200 may further include determining a second initial alignment measurement 160B of a second set of two adjacent segments (e.g., the third segment 102C and the fourth segment 102D) in the coupled position; comparing the second initial alignment measurement 160B to a second target alignment measurement 165B to determine a second adjustment amount; and adjusting at least one segment (e.g., the third segment 102C and/or the fourth segment 102D) of the second set of two adjacent segments based on the second adjustment amount.

Further, the exemplary method 200 may also include taking additional alignment measurements to determine if the tool 100 needs more adjustments until the target alignment measurement 165 is reached. For example, the method 200 may additionally include iteratively measuring and adjusting at least one of the plurality of segments 102 until the target alignment measurement 165 is achieved. This iterative method may include re-measuring an alignment measurement (e.g., the measurement corresponding to the initial alignment measurement 160) and re-adjusting the at least one of the plurality of segments 102, e.g., at least one of the first segment 102A and the second segment 102B, until the target alignment measurement 165 is reached, or until the alignment measurement is substantially equal to the target alignment measurement 165. By incrementally making adjustments to at least one of the first segment 102A and the second segment 102B, errors and/or incorrectly adjusted segments may be reduced. In certain embodiments, e.g., where the adjusting material 162 is a grinding, abrading, and/or eroding material, it will be appreciated that each incremental adjustment amount is smaller than the (actual) adjustment amount needed to achieve the target alignment measurement 165. The incremental adjustment amount may vary depending on the adjusting material 162; for example, where the adjusting material 162 is a milling material, a smaller incremental adjustment amount may be useful so as to not overly adjust the first segment 102A and/or the second segment 102B.

In still further embodiments, the method 200 may additionally and/or alternatively include loading the plurality of segments 102 prior to adjustment, as adjustments may affect how the plurality of segments 102 deform in relation to each other when loaded. Accordingly, representatively loading the plurality of segments 102 in between at least some adjustments to the plurality of segments 102 may reduce additional errors. It will be appreciated that loading the plurality of segments 102 may also be included as part of the iterative process described above.

Moreover, the method 200 may further be applied at a macro-level, e.g., where the first segment 102A and the second segment 102B are coupled together as a set and compared to another set of two or more segments, e.g., the third segment 102C and the fourth segment 102D, to determine the initial alignment measurement 160 and/or adjustment amount. These embodiments would allow for greater efficiency in the production of the tool 100 and may also keep production and labor costs at a minimum. In such an embodiment, the initial alignment measurement 160 may be a first initial alignment measurement 160A and the method 200 may further include determining a second initial alignment measurement 160B of a second set of two adjacent segments, e.g., the third segment 102C and the fourth segment 102D, in the coupled position; comparing the second initial alignment measurement 160B to a second target alignment measurement 165B to determine a second adjustment amount; and adjusting at least one of the third segment 102C and the fourth segment 102D based on the second adjustment amount. The second target alignment measurement 165B, in some embodiments, may be the same as the (first) target alignment measurement 165A. Alternatively, the second target alignment measurement 165B may be different from the first target alignment measurement 165A. Similarly, the second adjustment amount may or may not be the same as the (first) adjustment amount. It will be appreciated that the method 200 could be applied to multiple sets of adj acent segments, such as three sets of adjacent segments, four sets of adjacent segments, or even the entire plurality of segments 102.

It will be appreciated by those of ordinary skill in the art that method 200 may be automated and/or executed by one or more users to make these adjustments. In particular, the iterative process described above may be automated for efficiency.

Additionally, the method 200 may be altered such that an adjustment may be made to the tool 100 as a whole. For example, the plurality of segments 102 may be entirely assembled into tool 100, and a plurality of initial alignment measurements 160 may be taken, e.g., a plurality initial alignment measurement between each pair of adjacent segments in the plurality of segments 102. Similarly, there may be a plurality of target alignment measurements corresponding to each pair of adj acent segments in the plurality of segments 102. The method 200 may further include disassembling the tool 100 and adjusting each segment, e.g., the first segment 102A, according to the adjustment amount calculated for each pair of adjacent segments, e.g., the first segment 102A and the second segment 102B. The tool 100 may then be reassembled. In these embodiments, it may be helpful to assign unique markings and/or labels to identify each segment in the plurality of segments 102 in order to maintain the original order of the plurality segments 102. Further, in some additional and/or alternative embodiments, the tool 100 may be adjusted by selectively choosing a segment in the plurality segments 102 based on their initial alignment measurement 160 and assembling the tool 100 such that the plurality of segments 102 are arranged in a specific order, e.g., "selectively assembled."

In yet other embodiments, the method 200 may additionally and/or alternatively include measuring the shape of the tool 100 to determine an initial alignment measurement 160; comparing the initial alignment measurement 160 to a target alignment measurement 165 to determine an adjustment amount; and adjusting the tool 100 based on the adjustment amount. The shape of the tool 100 may be determined by measuring a radius of curvature and/or length of the tool 100. In one embodiment, the shape of the tool 100 is measured as a whole before adjustments are made to the tool 100. The adjustments may be made to at least one segment of the plurality of segments 102. In other non-limiting embodiments, the adjustments may be made to all of the plurality of segments 102 in the tool 100 and/or to the tool 100 as a whole. In one exemplary embodiment, the initial alignment measurement 160 may be determined using one or more images from a camera 111.

As mentioned previously, the method 200 may additionally and/or alternatively include loading the plurality of segments 102 prior to adjustment. Representatively loading the plurality of segments 102 in between at least some adjustments to the tool 100 may reduce additional errors. The adjustment amount for each segment, e.g., the first segment 102A, in the plurality of segments 102 may vary segment to segment in order to account for any gravity and/or other loading, such that the shape during the at least partial deployment of the tool 100 is the desired shape.

It will also be appreciated that the tool 100 may be used in any compatible machine across different industries. While reference is made herein with respect to turbofan engines 10 and gas turbine engines specifically, one of ordinary skill in the art will recognize that the inherent flexibility of the tool 100 allows for inspection and maintenance in different industrial machines of varying sizes.

Further aspects of the disclosure are provided by the subject matter of the following clauses:
A method for adjusting a tool insertable into a cavity of a machine comprising a plurality of segments, the method comprising: determining an initial alignment measurement of at least a portion of the tool; comparing the initial alignment measurement to a target alignment measurement to determine an adjustment amount; and adjusting at least one of the plurality of segments based on the adjustment amount.

The method of any preceding clause, wherein a first segment of the plurality of segments is adjacent to a second segment of the plurality of segments, wherein the first segment and the second segment are moveable between a bent position and a coupled position, and wherein determining an initial alignment measurement of at least a portion of the tool comprises determining an initial alignment measurement of the first segment and the second segment in the coupled position.

The method of any preceding clause, wherein at least one of the first segment and the second segment comprises or defines a guide feature, a drive feature, a line guide, or a combination thereof, and wherein the first segment comprises: a unique dimensional measurement affecting a fit tolerance with the second segment when in the coupled position, and wherein at least one of the first segment and the second segment at least partially comprises an adjustable material.

The method of any preceding clause, wherein adjusting at least one of the plurality of segments comprises at least one of removing material from and adding material to at least one of the first segment and the second segment.

The method of any preceding clause, wherein determining the initial alignment measurement of the first segment and the second segment in the coupled position comprises determining an angle measurement between the first segment and the second segment, and wherein the target alignment measurement is a target angle between the first segment and the second segment in the coupled position.

The method of any preceding clause, wherein determining the initial alignment measurement of the first segment and the second segment comprises: determining a first initial alignment measurement across an alignment feature, wherein the alignment feature extends between the first segment and the second segment; and determining a second initial alignment measurement between the first segment and the second segment along an edge opposite of the alignment feature.

The method of any preceding clause, wherein determining the initial alignment measurement of the first segment and the second segment comprises: determining a first initial alignment measurement across an alignment feature, wherein the alignment feature extends between the first segment and the second segment; and determining a second initial alignment measurement between the first segment and the second segment along an edge opposite of the alignment feature.

The method of any preceding clause, wherein determining the initial alignment measurement of the first segment and the second segment comprises: determining a first initial alignment measurement across an alignment feature, wherein the alignment feature extends between the first segment and the second segment; and determining a second initial alignment measurement between the first segment and the second segment along an edge opposite of the alignment feature.

The method of any preceding clause, wherein the first segment and the second segment define a first set of two adjacent segments and the method further comprises: determining a second initial alignment measurement of a second set of two adjacent segments in the coupled position; comparing the second initial alignment measurement to a second target alignment measurement to determine a second adjustment amount; and adjusting at least one segment of the second set of two adjacent segments based on the second adjustment amount.

The method of any preceding clause, the method further comprising: iteratively measuring and adjusting the at least one of the plurality of segments until the target alignment measurement is achieved.

The method of any preceding clause, further comprising: loading the plurality of segments to a representative working load prior to determining the initial alignment measurement.

A method for adjusting a tool, the method comprising: measuring a shape of the tool to determine an initial alignment measurement, wherein the tool is insertable into a cavity of a machine, the tool comprising a plurality of segments and wherein two adjacent segments of the plurality of segments are moveable between a bent position and a coupled position; comparing the initial alignment measurement to a target alignment measurement to determine an adjustment amount; and adjusting the tool based on the adjustment amount.

The method of any preceding clause, wherein measuring the shape of the tool further comprises: measuring a radius of curvature, a length, or both, of the tool prior to adjusting the tool based on the adjustment amount.

The method of any preceding clause, the method further comprising: loading the plurality of segments to a representative working load prior to determining the initial alignment measurement.

A tool for inserting into a cavity of a machine, the tool comprising: a plurality of segments moveably coupled, wherein one or more of the plurality of segments comprises a unique dimensional measurement affecting a fit tolerance.

The tool of any preceding clause, wherein a first segment of the plurality of segments is moveable relative to a second segment between a bent position and a coupled position, wherein the first segment is adjacent to the second segment.

The tool of any preceding clause, wherein the first segment further comprises a shell at least partially surrounding the core, wherein the core of the first segment comprises a first material, and wherein the shell comprises a second material.

The tool of any preceding clause, wherein at least one of the first material and the second material is an adjustable material.

The tool of any preceding clause, wherein the first segment further comprises a third material and wherein the third material is an adjustable material.

The tool of any preceding clause, wherein at least a portion of the tool defines an initial alignment measurement.

The tool of any preceding clause, wherein the tool is at least partially adjusted to a target alignment measurement and wherein the initial alignment measurement is different from the target alignment measurement.

The tool of any preceding clause, wherein the adjustable material is removed from the at least one of the first segment and the second segment, added to at least one of the first segment and the second segment, or both, to reach the target alignment measurement.

A method for adjusting a tool comprising a plurality of segments, the method comprising: determining an initial alignment measurement of at least a portion of the tool, wherein the tool is insertable into a cavity of a machine; comparing the initial alignment measurement to a target alignment measurement to determine an adjustment amount; and adjusting at least one of the plurality of segments based on the adjustment amount.

The method of any preceding clause, wherein a first segment of the plurality of segments is adjacent to a second segment of the plurality of segments, wherein the first segment and the second segment are moveable between a bent position and a coupled position, and wherein determining the initial alignment measurement of at least a portion of the tool comprises determining the initial alignment measurement of the first segment and the second segment in the coupled position.

The method of any preceding clause, wherein at least one of the first segment and the second segment comprises or defines a guide feature, a drive feature, a line guide, or a combination thereof, and wherein the first segment comprises: a unique dimensional measurement affecting a fit tolerance with the second segment when in the coupled position, and wherein at least one of the first segment and the second segment at least partially comprises an adjustable material.

The method of any preceding clause, wherein adjusting at least one of the plurality of segments comprises at least one of removing material from and adding material to at least one of the first segment and the second segment.

The method of any preceding clause, wherein determining the initial alignment measurement of the first segment and the second segment in the coupled position comprises determining an angle measurement between the first segment and the second segment, and wherein the target alignment measurement is a target angle between the first segment and the second segment in the coupled position.

The method of any preceding clause, wherein determining the initial alignment measurement of the first segment and the second segment comprises: determining a first initial alignment measurement across an alignment feature, wherein the alignment feature extends between the first segment and the second segment; and determining a second initial alignment measurement between the first segment and the second segment along an edge opposite of the alignment feature.

The method of any preceding clause, wherein the first segment and the second segment define a first set of two adjacent segments and the method further comprises: determining a second initial alignment measurement of a second set of two adjacent segments in the coupled position; comparing the second initial alignment measurement to a second target alignment measurement to determine a second adjustment amount; and adjusting at least one segment of the second set of two adjacent segments based on the second adjustment amount.

The method of any preceding clause, the method further comprising: iteratively measuring and adjusting the at least one of the plurality of segments until the target alignment measurement is achieved.

The method of any preceding clause, further comprising: loading the plurality of segments to a representative working load prior to determining the initial alignment measurement.

A method for adjusting a tool, the method comprising: measuring a shape of the tool to determine an initial alignment measurement, wherein the tool is insertable into a cavity of a machine, the tool comprising a plurality of segments and wherein two adjacent segments of the plurality of segments are moveable between a bent position and a coupled position; comparing the initial alignment measurement to a target alignment measurement to determine an adjustment amount; and adjusting the tool based on the adjustment amount.

The method of any preceding clause, wherein measuring the shape of the tool further comprises: measuring a radius of curvature, a length, or both, of the tool prior to adjusting the tool based on the adjustment amount.

The method of any preceding clause, the method further comprising: loading the plurality of segments to a representative working load prior to determining the initial alignment measurement.

A tool for inserting into a cavity of a machine, the tool comprising: a plurality of segments moveably coupled, wherein one or more of the plurality of segments comprises a unique dimensional measurement affecting a fit tolerance.

The tool of any preceding clause, wherein a first segment of the plurality of segments is moveable relative to a second segment between a bent position and a coupled position, wherein the first segment is adjacent to the second segment.

The tool of any preceding clause, wherein the first segment further comprises a shell at least partially surrounding the core, wherein the core of the first segment comprises a first material, and wherein the shell comprises a second material.

The tool of any preceding clause, wherein at least one of the first material and the second material is an adjustable material.

The tool of any preceding clause, wherein the first segment further comprises a third material and wherein the third material is an adjustable material.

The tool of any preceding clause, wherein at least a portion of the tool defines an initial alignment measurement.

The tool of any preceding clause, wherein the tool is at least partially adjusted to a target alignment measurement and wherein the initial alignment measurement is different from the target alignment measurement.

The tool of any preceding clause, wherein the adjustable material is removed from the at least one of the first segment and the second segment, added to at least one of the first segment and the second segment, or both, to reach the target alignment measurement.

This written description uses examples to disclose the present disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they include structural elements that do not differ from the literal language of the claims, or if they include equivalent structural elements with insubstantial differences from the literal languages of the claims.

## Claims

1. A method (200) for adjusting a tool (100) insertable into a cavity of a machine comprising a plurality of segments, wherein two adjacent segments (102) are moveable between a bent position and a coupled position, the method (200) comprising:
determining an initial alignment measurement (160) of the two adjacent segments (102) in the coupled position;
comparing the initial alignment measurement (160) to a target alignment measurement (165) to determine an adjustment amount; and
adjusting at least one of the two adjacent segments (102) based on the adjustment amount.

2. The method (200) of claim 1, wherein one or more of the plurality of segments comprises:
a unique dimensional measurement affecting a fit tolerance with the adjacent segment (102) when in the coupled position, and
wherein each segment (102) at least partially comprises an adjustable material (162).

3. The method (200) of any preceding claim, wherein adjusting at least one of the two adjacent segments (102) comprises at least one of removing material (162) from and adding material (162) to at least one of the two adjacent segments (102).

4. The method (200) of any preceding claim, further comprising:
loading the plurality of segments to a representative working load prior to determining the initial alignment measurement (160).

5. The method (200) of any preceding claim, wherein determining the initial alignment measurement (160) of the two adjacent segments (102) comprises determining an angle measurement between the two adjacent segments (102) in the coupled position, and wherein the target alignment measurement (165) is a target angle between the two adjacent segments (102) in the coupled position.

6. The method (200) of any preceding claim, wherein determining the initial alignment measurement (160) of the two adjacent segments (102) comprises:
determining a first initial alignment measurement (160) across an alignment feature (158), wherein the alignment feature (158) extends between the two adjacent segments (102); and
determining a second initial alignment measurement (160) across a bottom of the two adjacent segments (102).

7. The method (200) of any preceding claim, the method (200) further comprising:
determining a second initial alignment measurement (160) of a second set of two adjacent segments (102) in the coupled position;
comparing the second initial alignment measurement (160) to a second target alignment measurement (165) to determine a second adjustment amount; and
adjusting at least one of the second set of two adjacent segments (102) based on the second adjustment amount.

8. The method (200) of any preceding claim, the method (200) further comprising:
iteratively measuring and adjusting the at least one of the two adjacent segments (102) until the target alignment measurement (165) is achieved.

9. A tool (100) for inserting into a cavity of a machine, the tool (100) comprising:
a plurality of segments moveably coupled to one another, each of the plurality of segments moveable relative to an adjacent segment (102) between a bent position and a coupled position,
wherein one or more of the plurality of segments comprises a unique dimensional measurement affecting a fit tolerance with the adjacent segment (102) when in the coupled position, and
wherein each segment (102) at least partially comprises an adjustable material (162).

10. The tool (100) of claim 9, wherein a core (140) of a first segment (102) is configured to abut a core (140) of a forward adjacent segment (102A) and a core (140) of an aft-adjacent segment (102B).

11. The tool (100) of claim 10, wherein the first segment (102) further comprises a shell (142) at least partially surrounding the core (140), wherein the core (140) of the first segment (102) comprises a first material, and wherein the shell (142) comprises a second material.

12. The tool (100) of claim 11, wherein at least one of the first material and the
second
material is the adjustable material (162).

13. The tool (100) of any of claims 9 to 12, wherein at least one segment (102) in the plurality of segments defines an initial alignment measurement (160) in the coupled position.

14. The tool (100) of claim 13, wherein the adjustable material (162) can be at least partially adjusted to a target alignment measurement (165) defined between the at least one segment (102) and the adjacent segment (102) when in the coupled position, wherein the initial alignment measurement (160) is different from the target alignment measurement (165).

15. The tool (100) of claim 14, wherein the adjustable material (162) is removed from the at least one segment (102), added to the at least one segment (102), or both, to reach the target alignment measurement (165).
